# EUROPEAN PATENT APPLICATION

(11) **EP 3 818 936 A1**
(43) Date of publication of application: **12.05.2021**
(21) Application number: 20203991.3
(22) Date of filing: 27.10.2020
(51) Int. Cl.: A61B 5/282, A61B 5/26

(54) **BIOMETRIC DEVICE FOR THE HAND**

(30) Priority: 07.11.2019 TR 201917324
(71) Applicant: Arçelik Anonim Sirketi, 34445 Istanbul (TR)
(72) Inventor: CAKIN, Ilgaz, 34445 Istanbul (TR); HALATOGLU, Ugur, 34950 ISTANBUL (TR); TEKIN, Mehmet Ercan, 39450 ISTANBUL (TR)

(57) **Abstract**

The present invention relates to a biometric device (1) for measuring physiological parameters from a hand (A) of a patient. The biometric device (1) comprises a handle (2), an electrode (3) disposed on the handle (2), for taking electrical measurements from the hand (A), and an inflatable cuff (4). The invention further relates to a health kiosk and a fitness equipment comprising the biometric device (1).

## Description

The present invention relates to a biometric device for measuring physiological parameters such as electrocardiogram waveforms from a hand of a patient.

Developments in sensor technologies and ergonomic designs allow extensive application of biometrics technology, among others, in terms of health monitoring. Physiological measurements can easily be done by means of electrodes and required circuitry placed on equipment easily accessible in daily life. Electrocardiography (ECG) measurements from hands can be taken via steering wheel, handles on health kiosks or sport equipment designed accordingly. These devices help people conveniently monitor their heart functioning, control levels of fat burning, and/or diagnose potential problems regarding heart rhythm disorders, even without assistance of professional health personnel.

In ECG measurement systems, ECG signals are transmitted from electrodes to sensors, which in turn are processed by computing devices to obtain ECG waveforms. Even a marginal displacement of the hand with respect to electrodes on a handle, a change in contact surface or variations in the contact force thereof results in artifacts in the signals received from the subject. Distorted pulsation signals undermine the reliability of measurements and may even make the measurements unacceptable from a medical perspective.

In the state of the art, United States patent application US20170188846A1 discloses a handheld device for measuring vital signs and parameters from human body. The device comprises inflatable cuffs to engage with a patient's wrist and take pressure waveform measurements therefrom.

United States patent application US5727559 discloses a method and device for increasing hand vascular resistance during blood pressure measurement. Back of a hand is pressed against a board by inflating a bladder over the palm of the hand, and blood pressure measurements are taken from the wrist of the patient.

The aim of the present invention is the realization of a biometric device for measuring physiological parameters such as electrocardiogram waveforms from a hand of a patient, wherein the quality of signals received from the patient is increased. More particularly, it is a purpose of the present invention to prevent artifacts in signals measured from palm of a user via electrodes and obtain reliable signals and results of medical quality. The invention proposes a biometric device wherein, during measurements, the hand of the patient is prevented from moving with respect to a handle comprising the electrodes, and the contact area and contact force between the hand and the handle is fixed. In order to improve the quality of measurements, the biometric device also aims to maximise the contact area between the hand and the handle. Accordingly, with the proposed solution, it is possible to easily take ECG measurements of medical reliability and quality, without requiring professional medical assistance or use of patch electrodes.

The aim of the present invention is achieved by the biometric device explicated in claim 1, the health kiosk explicated in claim 13, and the fitness equipment explicated in claim 15. Further achievements have been attained by the subject-matters respectively defined in the dependent claims.

The biometric device for measuring physiological parameters from a hand of a patient comprises a handle, an electrode disposed on the handle for taking electrical measurements from the hand, and an inflatable cuff.

In a preferred embodiment, the cuff is disposed on the handle and the cuff is arranged to, when inflated, compress a hand gripping the handle towards the handle. The cuff may be arranged to, when inflated, restrict the movement of the hand gripping the handle with respect to the handle. By pressing the gripping hand towards the handle and restricting the movement of the hand, an accurate and high-quality signal measurement is made.

The cuff may comprise an air bladder, preferably made of elastic material. The air bladder, and hence the cuff, may be inflated by delivering air into the air bladder.

The cuff may bridge over the electrode such that an opening to receive the hand is formed between the handle and the cuff. The opening has a suitable for receiving at least one hand. However, the opening may be wide enough to receive two hands. For example, the cuff is arc shaped. When inflated, the cuff may have a concave inner surface for contacting the back of a hand gripping the handle. The concave surface is preferably designed to better fit upon the back of the hand gripping the handle, in order to further improve stability of hand on the handle. In some embodiments, the handle comprises at least one indent to receive fingers of the hand gripping the handle, which helps a better and more steady gripping of the hand.

The biometric device may further comprise an inflator for inflating the cuff. The biometric device may also comprise means for controlling the inflator, such as a start/stop button to start and stop inflation of the cuff.

In preferred embodiments, the measured physiological parameters relate to heartbeat induced pulsations and may comprise an electrocardiogram (ECG) waveform.

According to an aspect of the invention, there is provided a health kiosk comprising a biometric device in accordance with the foregoing description.

According to another aspect of the invention, there is provided a fitness equipment comprising a biometric device in accordance with the foregoing description.

The biometric device realized in order to attain the aim of the present invention is illustrated in the attached figures, where:
Figure 1 is a schematic illustration of the biometric device according to an embodiment, wherein the cuff is not inflated;
Figure 2 is a schematic illustration of the biometric device according to an embodiment, wherein the cuff is inflated.

The elements illustrated in the figures are numbered as follows:
1. Biometric device
2. Handle
3. Electrode
4. Cuff

### A. Hand

The biometric device (1) for measuring physiological parameters from a hand (A) of a patient comprises a handle (2), an electrode (3), and an inflatable cuff (4). One or more electrodes (3) may be disposed on the handle (2). The electrode (3) is arranged to take electrical measurements from the hand (A) while the hand (A) is in contact with the handle (2).

In a preferred embodiment, the cuff (4) is disposed on the handle (2) and is arranged to, when inflated, compress a hand (A) gripping the handle (2) towards the handle (2). When inflated, fully or sufficiently, the cuff (4) may thus restrict or even obstruct the movement of the hand (A) gripping the handle (2) with respect to the handle (2).

In operation, the biometric device (1) is electrically powered, for example, by means of a power chord from a power outlet or a battery (not shown in figures). The biometric device (1) comprises or is coupled to at least one sensor. The signals detected by the electrode (3) based on heartbeat induced pulsations of the patient are received by the sensor. The biometric device (1) comprises or coupled to a processing resource where sensed parameters are received from the at least one sensor and processed to obtain data relating to measured physiological parameters, such as ECG waveforms. Said physiological data may be transmitted to a remote computing device and/or displayed on a display means connected to the biometric device. Physiological readings obtained by the biometric device may be, for example, forwarded to a doctor for analysis and/or transmitted to a mobile computing device such as a laptop or a mobile phone.

The biometric device (1) may be arranged to accommodate one hand or two hands at a time.

According to an embodiment, the cuff (4) comprises at least one air bladder. The air bladder is preferably made of elastic material, such as plastic or flexible fabric. In this embodiment, the cuff (4) is inflated by delivering air pressure into the air bladder, hence inflating the air bladder. The cuff (4) may also be deflated by discharging the air in the air bladder. The cuff (4) may further comprise a housing to accommodate the air bladder.

In preferred embodiments, the cuff (4) is arranged on the handle (2) such that the cuff at least partly bridges over the area of the handle (2) wherein the electrode (3) is disposed. In this manner, an opening to receive the hand (A) is formed between the handle (2) and the cuff (4). Said opening may be at least partially enclosed by an inner surface of the cuff (4), facing towards the handle (2), and the handle (2) surface comprising at least one electrode (3). As a result, when a patient places his/her hand in the opening in a position touching the electrode (3) on the handle (2), an ECG measurement from the hand (A) can be taken. The cuff (4) may be in the form of a bar or strap and may be arc shaped. The cuff (4) may have a U-shaped or L-shaped form. The cuff (4) is mechanically connected to the handle from at least one point, preferably from two points.

At least in its inflated position, the cuff (4) may have a concave inner surface. The inner surface refers to the surface facing the handle (2). When the cuff (4) is inflated, the inner surface at least partially contacts and presses on a hand (A) that is placed in the opening between the cuff (4) and the handle (2). When the hand (A) grips or grasps the handle (2), for example, the inner surface of the inflated cuff (4) is contact with the back of the hand. The concavity therefore helps establishing a wider contact surface with the back of the hand (A) and more effectively fixes the gripping hand (A) on the handle (2). In embodiments where the cuff (4) comprises an inflatable air bladder as described above, the inner surface may be that of the air bladder.

In accordance with the purposes of the invention, the handle (2) may comprise a finger engagement structure, such as one or more indents and/or one or more grooves to receive fingers of the hand (A) gripping the handle. The handle is preferably in the form of a bar and may have a circular, elliptical or rounded rectangular cross section.

The cuff (4) can be inflated manually or automatically by an inflator delivering air into an air chamber comprised in the cuff (4), for example into the air bladder. Similarly, the cuff (4) can be deflated manually or automatically by a deflator discharging air from said air chamber. The inflator and deflator can be embodied on a single module. Thus, the inflator may also be operated as a deflator. The biometric device (1) comprises and/or is arranged to be connected to the inflator. The inflator is preferably controlled by a controlling means. Means for controlling the inflator may be in the form of a switch, button, and a soft key. The control provided via said means comprises one or more of starting inflation, stopping inflation, starting deflation, and stopping deflation of the cuff (4). Means for controlling the inflator may be disposed on the biometric device (1). Accordingly, a patient may easily control inflation/deflation of the cuff (4) in order to measure physiological parameters from one of his/her hands by using the biometric device (1), without any help from another person. Physiological parameters that can be measured by the biometric device comprise electrocardiogram (ECG) waveforms.

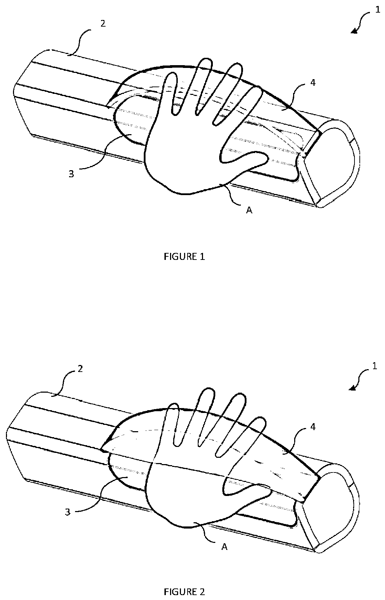

Now an exemplary use case for taking ECG measurements from one hand (A) by using the biometric device (1) will be described. The cuff (4) is in, or is brought to, a state that it is at least partially deflated, so that a user (i.e. patient) can conveniently put his hand (A) through an opening between the inner surface of the cuff (4) and the handle (2). The patient places his hand through the opening (A) on the handle (2) such that palm of the hand (A) touches the electrodes (3), preferably by grasping the handle (2) (Figure 1). Before starting the measurement, the cuff (4) is inflated sufficiently to fasten the hand (A) on the handle (2). The hand (A) is thus fixed with respect to the electrodes (3) (Figure 2). Then ECG measurements begin, taking electrical signals from the fastened hand (A) via electrodes (3). The hand (A) tightly grips the handle (4) and it is at the same time compressed by the inflated cuff (4). As a result, displacement of the hand (A) relative to the handle (4) and the electrode (3), and therefore possible artifacts in the obtained signal are prevented. Once the measurement is complete, the cuff (4) is at least partially deflated to release the hand (A).

Present invention extends a health kiosk comprising a biometric device (1) in accordance with any one of the embodiments described above. The invention also extends to a fitness equipment comprising the biometric device (1) in accordance with any one of the embodiments described above. The health kiosk or the fitness equipment may comprise more than one biometric device (1), for example one or more pairs of biometric devices (1).

In view of technical features and details explained above, the present invention proposes a biometric device (1) wherein the process of taking physiological measurements from a patient's hand is facilitated. Furthermore, the quality of obtained signals and therefore reliability of measurements are improved.

## Claims

1. A biometric device (1) for measuring physiological parameters from a hand (A) of a patient, comprising a handle (2), an electrode (3) disposed on the handle (2) for taking electrical measurements from the hand (A), and an inflatable cuff (4); **characterised in that** the cuff (4) is disposed on the handle (2) and arranged to, when inflated, compress a hand (A) gripping the handle (2) towards the handle (2).

2. The biometric device (1) according to claim 1, wherein the cuff (4) is arranged to, when inflated, restrict the movement of the hand (A) gripping the handle (2) with respect to the handle (2).

3. The biometric device (1) according to claim 1 or 2, wherein the cuff (4) comprises an air bladder.

4. The biometric device (1) according to any one of preceding claims, wherein the cuff (4) bridges over the electrode (3) such that an opening to receive the hand (A) is formed between the handle (2) and the cuff (4).

5. The biometric device (1) according to any one of preceding claims, wherein the cuff (4) is arc shaped.

6. The biometric device (1) according to any one of preceding claims, wherein the cuff (4), when inflated, has a concave inner surface for contacting the back of a hand (A) gripping the handle (2).

7. The biometric device (1) according to any one of preceding claims, wherein the handle (2) comprises at least one indent to receive fingers of the hand (A) gripping the handle.

8. The biometric device (1) according to any one of preceding claims, wherein the biometric device (1) further comprises an inflator for inflating the cuff (4).

9. The biometric device (1) according to claim 8, wherein the biometric device (8) further comprises means for controlling the inflator.

10. The biometric device (1) according to any one of preceding claims, wherein the measured physiological parameters comprise an electrocardiogram (ECG) waveform.

11. A health kiosk comprising the biometric device (1) according to any one of preceding claims.

12. A fitness equipment comprising the biometric device (1) according to any one of claims 1 to 10.
